# EUROPEAN PATENT SPECIFICATION

(11) **EP 3 787 731 B1**
(45) Date of publication and mention of the grant of the patent: **21.06.2023**
(21) Application number: 18917355.2
(22) Date of filing: 25.06.2018
(51) Int. Cl.: A61M 25/10, A61L 29/08, A61L 29/16

(54) **PROCESS FOR PLEATING AND FOLDING DRUG COATED BALLOON**
VERFAHREN ZUM FALTEN EINES MEDIKAMENTENBESCHICHTETEN BALLONS
PROCÉDÉ DE PLISSAGE ET DE PLIAGE D'UN BALLONNET REVÊTU DE MÉDICAMENT

(30) Priority: 02.05.2018 IN 201821016579
(43) Date of publication of application: 10.03.2021
(73) Proprietor: Meril Life Sciences Pvt Ltd, Gujarat, Vapi 396191 (IN)
(72) Inventor: KOTHWALA, Deveshkumar Mahendralal, Surat, Gujarat 395003 (IN); VYAS, Rajnikant Gandalal, Mumbai, Powai 400076 (IN); MINOCHA, Dr. Pramod Kumar, Vapi, Gujarat 396191 (IN)
(74) Representative: Forrest, Stuart
(86) International application number: PCT/IN2018/050415
(87) International publication number: WO 2019/211861

(56) References cited:
- EP-A1- 3 238 773
- US-A1- 2005 261 723
- US-A1- 2008 243 221
- US-A1- 2011 099 789
- US-A1- 2012 065 583
- US-A1- 2012 128 863

## Description

### FIELD OF INVENTION

The present invention relates to a process, more specifically the present invention relates to a pleating and folding process for a drug coated balloon.

### BACKGROUND

Peripheral artery disease (PAD) is a disease in which plaque builds up in the arteries that carry blood to head, organs and limbs. PAD usually affects the arteries of the legs however it can also affect the arteries that carry blood from heart to head, kidneys, arms, and stomach. A drug coated balloon may be used for the treatment of peripheral artery disease. A drug coated balloon (DCB) is a conventional semi-compliant angioplasty balloon covered with an anti-proliferative drug which is released into the vessel wall during inflation of the balloon.

The wrapping/pleating/folding process is carried out to protect the coating applied on the surface of the drug coated balloon. The wrapping/pleating/folding process is carried out to reduce the profile of the drug coated balloon for easy delivery of the balloon catheter.

The processes presently available for wrapping/pleating/folding result in loss of the anti-proliferative drug. These processes also result in cracks being developed in the coating of the drug coated balloon.

Therefore, the process which deals with the above mentioned drawbacks is required to be devised. Throughout the description, pressure are defined in pounds per square inch (psi). 1psi=0,07atm=6,89kPa

### SUMMARY

The present invention as defined in the claims discloses a pleating and folding process of a drug coated balloon. A balloon is firstly inflated and an outer surface is coated with one or more layers of therapeutic agents and/or excipients to form a drug coated balloon.

The drug coated balloon, is subjected to pleating at a temperature from the range of 25°-60°C and at a pressure from the range of 25-35 psi.

The drug coated balloon is then subjected to folding in a predefined direction at a pressure ranging from 80-100 psi and a dwell time ranging of 70-110 seconds.

After the pleating and folding processes are completed, a protective sheath is mounted over the outer surface of the drug coated balloon to hold the pleats of the folded balloon.

After mounting, the heat setting process takes place at a temperature of 30-60°C which results in the balloon having a reduced profile ranging from 60% to 70% of the initial balloon diameter.

### BRIEF DESCRIPTION OF THE DRAWINGS

The summary above, as well as the following detailed description of illustrative embodiments, is better understood when read in conjunction with the appended drawings. For the purpose of illustrating the present disclosure, exemplary constructions of the disclosure are shown in the drawings. However, the disclosure is not limited to specific methods and instrumentalities disclosed herein. Moreover, those in the art will understand that the drawings are not to scale.
Fig. 1 represents a drug coated balloon catheter in accordance with an embodiment of the present invention.
Fig. 2 (a) illustrates a flow chart depicting the pleating process of the drug coated balloon in accordance with an embodiment of the present invention.
Fig. 2 (b) illustrates a flow chart depicting the folding process of the drug coated balloon in accordance with an embodiment of the present invention.
Fig.3 represents the side view and front view of a pleated and folded drug coated balloon.

### DETAILED DESCRIPTION OF THE DRAWINGS

Prior to describing the invention in detail, definitions of certain words or phrases used throughout this patent document will be defined: the terms "include" and "comprise", as well as derivatives thereof, mean inclusion without limitation; the term "or" is inclusive, meaning and/or; the phrases "coupled with" and "associated therewith", as well as derivatives thereof, may mean to include, be included within, interconnect with, contain, be contained within, connect to or with, couple to or with, be communicable with, cooperate with, interleave, juxtapose, be proximate to, be bound to or with, have a property of, or the like; Definitions of certain words and phrases are provided throughout this patent document, and those of ordinary skill in the art will understand that such definitions apply in many, if not most, instances to prior as well as future uses of such defined words and phrases.

Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In accordance with the present disclosure, the process for pleating and folding a drug coated balloon is disclosed. The pleating and folding is done after the balloon is coated with one or more layers of therapeutic agent and/or excipients. The pleating and folding is done after the coating process to ensure that the therapeutic agent remains intact within the pleats and folds formed on the drug coated balloon. The coating integrity is maintained after the pleating and folding process of the drug coated balloon is completed. The pleating and folding protects the coating layer(s) on the drug coated balloon. The pleating and folding process ensures minimal cracking on the coating surface of the drug coated balloon.

In an embodiment, pleating and folding is done in a pleating and folding room. The temperature and humidity of the pleating and folding room influence the pleating and folding process. The temperature of the pleating and folding room ranges from 19-25°C. The relative humidity of the pleating and folding room ranges from ranges from 40-60%.

In the invention, the drug coated balloon is inserted inside a pleating station where pleats on the drug coated balloon are formed. Subsequently, the drug coated balloon is folded inside a folding station. The drug coated balloon is then subjected to sterilization after the pleating and folding process is completed.

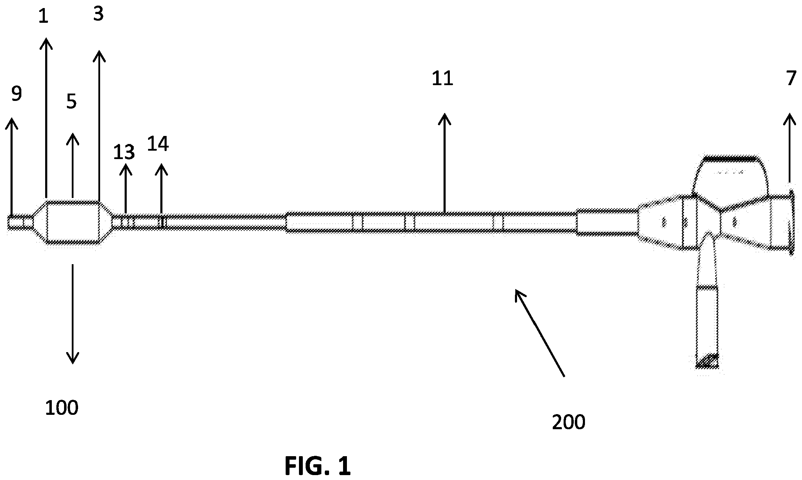

Now referring specifically to the drawings, Fig. 1 represents an exemplary schematic view of a drug coated balloon catheter 200. As represented, the drug coated balloon catheter 200 includes without limitation, a drug coated balloon 100, a distal end 9, a proximal end 7, a shaft 11 and one or more radiopaque marker bands 13 and 14.

The drug coated balloon 100 includes without limitation a balloon 10 coated with one or more layers of a therapeutic agent and/or excipients. The balloon 10 further includes a distal end 1, a proximal end 3 and an outer surface 5. In an embodiment, the distal end 1 and the proximal end 3 of the balloon 10 are tapered.

In an embodiment, the balloon 10 may be made of a polymeric material. The polymeric material may include without limitation nylon, pebax, polyethylene terephthalate, polyurethane etc.

The drug coated balloon 100 is mounted on the shaft 11 of the balloon catheter 200. The diameter of the drug coated balloon 100 before expansion ranges from 1.5-3.00mm depending on the size of the drug coated balloon. The drug coated balloon 100 may expand when an inflation fluid or inflation pressure is applied to the drug coated balloon 100 via a hub provided at the proximal end 7 of the balloon catheter 200. During balloon expansion at the treatment site, the distal end 1 and the proximal end 3 of the drug coated balloon 100 do not come in contact with an arterial tissue wall while the drug is transferred to the underlying tissue.

The drug coated balloon 100 may be, without limitation, a percutaneous transluminal angioplasty balloon (PTA), a percutaneous transluminal valvuloplasty (PTV), a percutaneous transluminal coronary angioplasty (PTCA balloon), a tapered balloon, a pediatric balloon, etc.

The shaft 11 is a hollow tubular structure. The shaft 11 may be made of, without limitation polymeric materials or metals. The shaft 11 has a hollow portion, a distal end and a proximal end. The hollow portion of shaft 11 extends from the proximal end to the distal end. The hollow portion provides a passage way for components like inner tube, inflation fluid, guide wire etc. The shaft 11 permits the movement of the guide wire.

The radiopaque marker bands 13 and 14 can be optionally provided on the catheter 200. As an example, the radiopaque marker bands 13 and 14 are placed near the distal end 9 of the catheter 200. Though in the context of the invention two radiopaque marker bands are described, it is possible that more or lesser number of makers may be used. The radiopaque marker bands 13 and 14 help to position the drug coated balloon 100 during deployment of the drug coated balloon 100 inside a diseased artery.

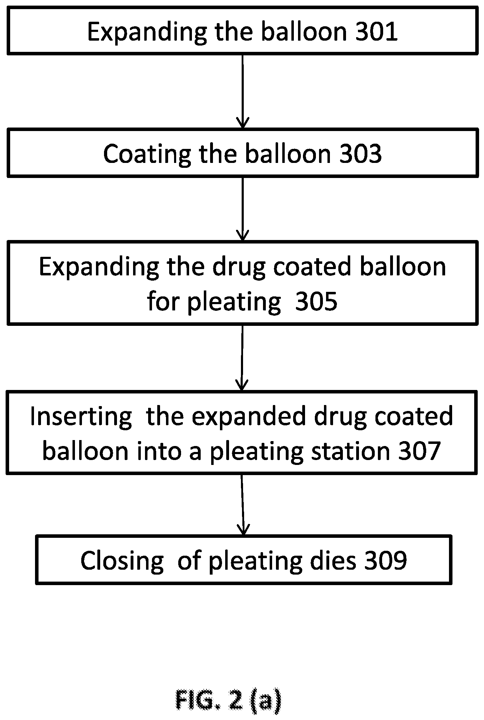

Fig. 2 (a) illustrates a flow chart depicting the pleating process of the drug coated balloon 100. The pleating process commences at step 301. The balloon 10 is fully expanded at a nominal pressure that ranges from 6- 8 atm depending on the size of the balloon 10. The complete expansion of the balloon 10 ensures even distribution of the drug over the balloon surface area. The purpose of completely expanding the balloon 10 is to mimic the inflated balloon condition at the treatment site. The diameter of the balloon 10 after expansion ranges from 3.00-10mm as per the dimension of the vessel at the treatment site.

At step 303, the coating process on the outer surface 5 of the balloon 10 is commenced. The coating on the outer surface 5 of the balloon 10 maybe done in the form of one or more layers. The coating composition may include a therapeutic agent and/or excipients. The therapeutic agent(s) may be selected from one or more of, without limitation, sirolimus, biolimus, zotarolimus, everolimus, paclitaxel, vinblastine, vindesine or vincristine. The excipients may include, without limitation binders, plasticizers, emulsifiers, salts, humectants, stabilizers. The optimum lubricity and wettability of the coating formulation utilized in the present invention results in reduced profile of the drug coated balloon 100 and also results in maintaining coating integrity of the drug coated balloon 100 after pleating and folding process.

The coating is applied before the pleating process to ensure uniform coating of the drug over the outer surface 5 of the balloon 10. Alternatively, but not forming part of the invention, the coating may be applied after the pleating process.

Further, material of pleating dies affects the coating integrity during pleating folding process.

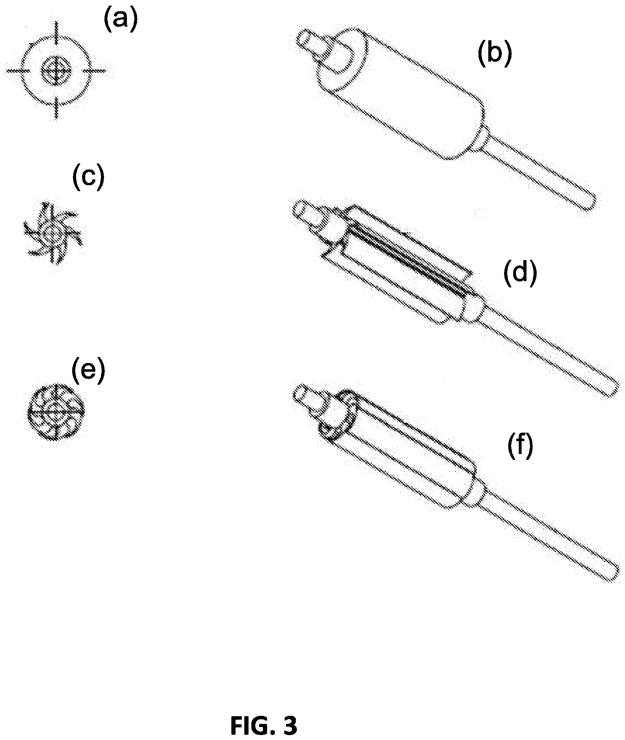

At step 305, the drug coated balloon 100 (as formed at step 303) is completely expanded at a pressure of for example, 30+/- 5 psi. Fig. 3 (a) and Fig. 3 (b) depict the initial front view and side view of the drug coated balloon 100 respectively.

At step 307, the expanded drug coated balloon 100 is inserted inside a pleating station. In an embodiment, the pleating station includes a plurality of pleating dies. In yet another embodiment, the material of pleating die is made up of stainless steel. The pleating dies may be heated to a temperature of 30°- 60°C more preferably 40°- 50°C. The selection of the temperature range may be an important and critical aspect for the pleating process since increase in temperature may cause drug degradation while lower temperature may not lead to formation of uniform pleats or the pleats formed may not retain their shape for a longer period of time. In an embodiment, the temperature range is selected depending upon the properties and characteristics of the coating materials. The expanded drug coated balloon 100 is inserted in a manner that the expanded surface of the drug coated balloon 100 comes in contact with the pleating dies. The pleating dies form pleats on the outer surface of the drug coated balloon when they come in contact with the outer surface.

The pleating dies may or may not be covered with a protective film. In an embodiment, the protective film is a Teflon film. The protective film protects the coating on the outer surface 5 of the expanded drug coated balloon 100 while achieving uniform pleating.

At step 309, the pleating dies are positioned close to the expanded surface of the drug coated balloon 100 such that once the pleating dies are turned on the pleating dies form pleats on the outer surface of the balloon. In an embodiment, six pleats are formed when the pleating dies are closed around the expanded drug coated balloon 100. The formation of six pleats on the drug coated balloon 100 helps in achieving a reduced crossing profile. The formation of six pleats on the drug coated balloon 100 also ensures minimal loss of the therapeutic agent as the therapeutic agent remains intact between the pleats of the drug coated balloon 100.

The dwell time for the pleating process ranges from 70-110 seconds more preferably between is 80-100 seconds. The length of the pleats formed on the drug coated balloon 100 is same as the length of the drug coated balloon 100 as pleating is done over the entire surface of the drug coated balloon. The length of drug coated balloon 100 ranges from 20.00- 300.00 mm.

It is to be noted that given the drug coated balloon 100 is completely expanded, the pleats formed on the outer surface are more pronounced (Fig. 3). Further, vacuum is applied to the drug coated balloon 100 which ensures that the pleated shape is retained during the folding step. The vacuum delay time ranges from 10-30 seconds more preferably 15-25 seconds. Fig. 3 (c) and Fig. 3 (d) depict the front view and side view of the pleated drug coated balloon 100 respectively.

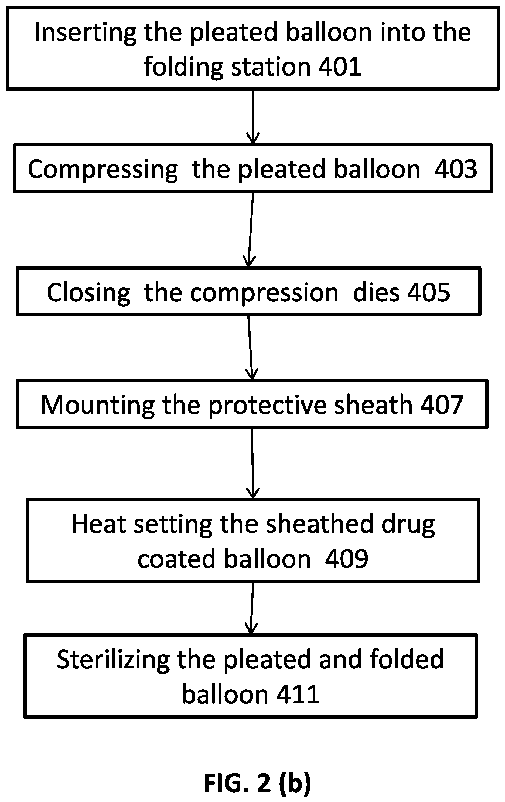

Fig. 2 (b) illustrates a flow chart depicting the folding process of the drug coated balloon 100. The folding process commences at step 401. The pleated drug coated balloon 100 is inserted inside the folding station. In an embodiment, the folding station comprises of a plurality of compression dies. The compression dies are heated to a temperature of 30-60°C more preferably 40- 50°C.

At step 403, the pleated drug coated balloon 100 is folded at a pressure of for example, 90+/- 10 psi.

At step 405, the compression dies are closed around the pleated drug coated balloon 100 while the vacuum is still applied. In an embodiment, the pleats of the drug coated balloon 100 are folded in a clockwise direction. In an embodiment, the drug coated balloon 100 is folded in such a pattern that when the drug coated balloon 100 is viewed from its distal end 1 by normal vision the drug coated balloon 100 appears six fluted in clockwise direction. The dwell time in which the folding process is completed ranges from 70-110 seconds more preferably between is 80-100 seconds.

The drug coated balloon 100 when subjected to pleating and folding process results in substantial reduction in profile of the drug coated balloon 100 Fig. 3 (e) and Fig. 3 (f) depict the front view and side view of the folded drug coated balloon 100 respectively.

The diameter of the drug coated balloon 100 after pleating and folding is reduced to approximately 60 % to 70 % of its initial diameter.

Post folding process as per the teachings of the present invention, the coating integrity of the drug coated balloon 100 remains intact. The pleating and folding area does not deform, or damage after the pleating and folding process is completed. The above mentioned features are achieved due to optimum temperature and optimum pleat/fold time.

At step 407, the drug coated balloon 100 is mounted with a protective sheath. In an embodiment, the protective sheath is made of, without limitation polytetrafluroethylene (PTFE), heat shrink PTFE, Nylon, polyetheretherketone (PEEK).

The protective sheath is mounted over the outer surface 5 of the drug coated balloon 100 within a period of 5-10 minutes after the folding process of the balloon is completed while the vacuum is still applied. The protective sheath is mounted on the drug coated balloon 100 to protect the drug layer coated on the outer surface 5 of the drug coated balloon 100 from any damage during handling, storage and shipping. The protective sheath also prevents recoil of the drug coated balloon 100. The protective sheath also helps in maintaining the reduced profile of the coated balloon.

At step 409, the sheathed drug coated balloon 100 is subjected to a heat set process. The coated and sheathed balloon 100 is heated at a temperature of 30-60°C to set the pleats formed over the drug coated balloon 100. The heat set process is carried out for a time period of for example, 6+/-3 minutes. The heat set process results in achieving a reduced profile of the drug coated balloon 100 ranging from 60-70% of initial the balloon diameter.

The heat set process further, evenly wraps the pleats in a clockwise direction thus ensuring uniform crossing profile of the drug coated balloon 100. The drug coated balloon 100 after the heat set process enhances the shape memory of pleats formed. There is no impact of heat setting process on the drug layer coated on the drug coated balloon 100. Also, there is no effect of the heat set process on the efficiency of the therapeutic drug coated on the drug coated balloon 100.

At step 411, the drug coated balloon 100 after pleating and folding is subjected to sterilization. The drug coated balloon 100 is sterilized by means of, without limitation ethylene oxide sterilization.

The present invention as described above can be explained with the help of following example which includes formulations A and B used for coating the balloon and pleating and folding parameters.

### Example 1:

**Table 1**

| **Coating components** | **Formulation A** | **Formulation B** |
|---|---|---|
| Paclitaxel (weight %) | 61.81 | 63.19 |
| Urea (weight %) | 11.32 | 31.94 |
| PEG200 (weight %) | 22.08 | 04.86 |
| Acetyl Tributyl Citrate (ATBC) (weight %) | 00.25 | - |
| Glycerol (weight %) | 04.52 | - |

| **Pleating process parameter** | | |
|---|---|---|
| Temperature (°C) | 30 ± 5 | 45 ± 5 |
| Dwell Time (seconds) | 90 ± 9 | 90 ± 9 |
| Balloon Pressure (psi) | 20 ± 5 | 30 ± 5 |
| Vacuum Delay (seconds) | 20 ± 5 | 20 ± 5 |

| **Folding process parameter** | | |
|---|---|---|
| Temperature (°C) | 30 ± 5 | 45 ± 5 |
| Dwell Time (seconds) | 90 ± 9 | 90 ± 9 |
| Pressure (psi) | 70 ± 10 | 90 ± 10 |

The balloon coated with formulation A was observed to have cracking and peeling of coating layer after pleating and folding process at above mentioned parameters. Also, the pleats formed did not retain their shape resulting in non-uniform folding process. The crossing profile is reduced only by 40% to 50% of the initial balloon diameter. On the other hand, balloon surface coated with formulation B is observed to have no surface damage after pleating folding process at the parameters specified in the table above. The increase in temperature during pleating process preserved the shape of pleats which further lead to uniform folding of the drug coated pleated balloon. The crossing profile is hence reduced by 60% to 70% of the initial balloon diameter.

The scope of the invention is only limited by the appended patent claims. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used.

## Claims

1. A method of folding a balloon, the method comprising:
a. inflating a balloon;
b. coating the balloon with one or more layers of therapeutic agents and/or excipients;
c. pleating the coated balloon at a temperature from the range of 25°-60°C and at a pressure from the range of 25-35 psi;
d. folding the pleated balloon in a predefined direction at a pressure 80-100 psi and a dwell time of 70-110 seconds;
e. mounting a sheath on the folded balloon to hold pleats of the folded balloon; and
f. heat setting the sheath and the folded balloon at a temperature of 30-60°C which results in the balloon having a reduced profile ranging 60% to 70% of initial balloon diameter.

2. The method of claim 1, wherein the pleating results in six pleats.

3. The method of claim 1, wherein the predefined direction comprises a clockwise direction.

4. The method of claim 1, wherein after the step of heat setting, the method comprises sterilizing the heat set balloon.

## Patentansprüche

1. Verfahren zum Zusammenlegen eines Ballons, wobei das Verfahren Folgendes umfasst:
a. Aufblasen eines Ballons,
b. Beschichten des Ballons mit einer oder mehreren Schichten von Therapeutika und/oder Hilfsstoffen;
c. Falten des beschichteten Ballons bei einer Temperatur aus dem Bereich von 25-60 °C und bei einem Druck aus dem Bereich von 25-35 psi;
d. Zusammenlegen des gefalteten Ballons in einer vorgegebenen Richtung bei einem Druck von 80-100 psi und einer Verweilzeit von 70-110 Sekunden;
e. Anbringen einer Hülle an dem zusammengelegten Ballon, um die Falten des zusammengelegten Ballons zu halten; und
f. Thermofixieren der Hülle und des zusammengelegten Ballons bei einer Temperatur von 30-60 °C, was dazu führt, dass der Ballon ein verringertes Profil im Bereich von 60 % bis 70 % des Ausgangsballondurchmessers aufweist.

2. Verfahren nach Anspruch 1, wobei das Falten sechs Falten ergibt.

3. Verfahren nach Anspruch 1, wobei die vorgegebene Richtung einen Uhrzeigersinn umfasst.

4. Verfahren nach Anspruch 1, wobei das Verfahren nach dem Schritt des Thermofixierens das Sterilisieren des thermofixierten Ballons umfasst.

## Revendications

1. Procédé de pliage d'un ballonnet, le procédé comprenant :
a. le gonflage d'un ballonnet ;
b. le revêtement du ballonnet avec une ou plusieurs couches d'agents thérapeutiques et/ou d'excipients ;
c. le plissage du ballonnet revêtu à une température dans la plage de 25 à 60 °C et à une pression dans la plage de 25 à 35 psi ;
d. le pliage du ballonnet plissé dans une direction prédéfinie à une pression de 80 à 100 psi et un temps de maintien de 70 à 110 secondes ;
e. le montage d'une gaine sur le ballonnet plié pour maintenir les plissures du ballonnet plié ; et
f. la thermofixation de la gaine et du ballonnet plié à une température de 30 à 60 °C qui a pour résultat que le ballonnet a un profil réduit allant de 60 % à 70 % du diamètre de ballonnet initial.

2. Procédé selon la revendication 1, dans lequel le plissage a six plissures pour résultat.

3. Procédé selon la revendication 1, dans lequel la direction prédéfinie comprend une direction dans le sens des aiguilles d'une montre.

4. Procédé selon la revendication 1, dans lequel après l'étape de thermofixation, le procédé comprend la stérilisation du ballonnet thermofixé.
